# EUROPEAN PATENT SPECIFICATION

(11) **EP 1 228 232 B1**
(45) Date of publication and mention of the grant of the patent: **17.01.2007**
(21) Application number: 00971756.2
(22) Date of filing: 02.11.2000
(51) Int. Cl.: C12N 15/86, C12N 15/12, C07K 14/47, A61K 48/00

(54) **RECOMBINANT SENDAI VIRUS VECTOR FOR INTRODUCING EXOGENOUS GENES TO AIRWAY EPITHELIA**
REKOMBINANTER SENDAIVIRUS-VEKTOR FÜR DIE EINFÜHRUNG VON FREMDGENEN INS EPITHEL DER ATEMWEGE
VECTEUR DU VIRUS DE SENDAI RECOMBINE SERVANT A INTRODUIRE DES GENES EXOGENES DANS L'EPITHELIUM DES VOIES RESPIRATOIRES

(30) Priority: 02.11.1999 US 163055 P; 17.12.1999 JP 35921899
(43) Date of publication of application: 07.08.2002
(73) Proprietor: Dnavec Research Inc., Tsukuba-shi, Ibaraki 305-0856 (JP)
(72) Inventor: YONEMITSU, Yoshikazu Kyushu University, Fukuoka 812-8582 (JP); HASEGAWA, Mamoru DNAVEC Research Inc., Ibaraki 305-0856 (JP); ALTON, Eric, WFW Imperial College School Medicine, Emmanuel Kaye Bldg Manresa Road (GB)
(74) Representative: Vossius & Partner
(86) International application number: PCT/JP2000/007737
(87) International publication number: WO 2001/032898

(56) References cited:
- YONEMITSU Y. ET AL.: "HVJ (Sendai virus)-cationic liposomes: A novel and potentially effective liposome-mediated technique for gene transfer to the airway epithelium." GENE THERAPY, vol. 4, no. 7, 1997, pages 631-638, XP000992923 ISSN: 0969-7128
- HASAN M. K. ET AL.: "CREATION OF AN INFECTIOUS RECOMBINANT SENDAI VIRUS EXPRESSING THE FIRELY LUCIFERASE GENE FROM THE 3' PROXIMAL FIRST LOCUS" JOURNAL OF GENERAL VIROLOGY, vol. 78, no. 11, 1997, pages 2813-2820, XP000886572 ISSN: 0022-1317
- SAEKI Y. ET AL.: "Development and characterization of cationic liposomes conjugated with HVJ (Sendai virus): reciprocal effect of cationic lipid for in vitro and in vivo gene transfer." HUMAN GENE THERAPY, vol. 8, 20 November 1997 (1997-11-20), pages 2133-2141, XP000992958
- SAKAI Y. ET AL.: "ACCOMMODATION OF FOREIGN GENES INTO THE SENDAI VIRUS GENOME: SIZES OF INSERTED GENES AND VIRAL REPLICATION" FEBS LETTERS, vol. 456, 6 August 1999 (1999-08-06), pages 221-226, XP000887286 ISSN: 0014-5793
- YONEMITSU Y. ET AL.: "Efficient gene transfer to airway epithelium using recombinant Sendai virus." NATURE BIOTECHNOLOGY, vol. 18, no. 9, September 2000 (2000-09), pages 970-973, XP002165781 ISSN: 1087-0156
- RETRIEVED FROM THE INTERNET: 'URL:http//www.serono-canada.com/english/so lution/ms/index.htm', * paragraph [000R] - paragraph [000W] *
- FERRARI S. ET AL: 'Recombinant Sendai Virus-Mediated CFTR cDNA Transfer' MOLECULAR THERAPY vol. 7, no. 5, May 2003, page S38

### SEQUENCE LISTING

<110> DNAVEC Research Inc.
<120> RECOMBINANT SENDAI VIRUS VECTOR FOR INTRODUCING EXOGENOUS GENES T0 AIRWAY EPITHELIA
<130> D3-105PCT
<140>
   <141>
<150> US 60/163,055
   <151> 1999-11-02
<150> JP 1999-359218
   <151> 1999-12-17
<160> 5
<170> PatentIn Ver. 2.1
<210> 1
   <211> 10
   <212> DNA
   <213> Artificial Sequence
<220>
   <223> Description of Artificial Sequence: artificially synthesized sequence
<400> 1
   ctttcaccct 10
<210> 2
   <211> 15
   <212> DNA
   <213> Artificial Sequence
<220>
   <223> Description of Artificial Sequence: artificially synthesized sequence
<400> 2
   tttttcttac tacgg 15
<210> 3
   <211> 18
   <212> DNA
   <213> Artificial Sequence
<220>
   <223> Description of Artificial Sequence: artificially synthesized sequence
<400> 3
   cggccgcaga tcttcacg 18
<210> 4
   <211> 51
   <212> DNA
   <213> Artificial Sequence
<220>
   <223> Description of Artificial Sequence: artificially synthesized sequence
<400> 4
   acttgcggcc gccaaagttc aatgcagagg tcgcctctgg aaaaggccag c 51
<210> 5
   <211> 76
   <212> DNA
   <213> Artificial Sequence
<220>
   <223> Description of Artificial Sequence: artificially synthesized sequence
<400> 5
   atccgcggcc gcgatgaact ttcaccctaa gtttttctta ctacggctaa agccttgtat 60
   cttgcacctc ttcttc                                                 76

## Description

### Technical Field

The present invention relates to composition comprising a recombinant Sendai virus vector suitable for introducing exogenous genes to airway epithelia and suitable for a method for introducing exogenous genes using the vector.

### Background Art

With the advent of molecular cloning techniques, an expanding array of genes with mutations responsible for important human diseases have been identified and isolated. Absent or mutated genes in human patients can be replaced by *ex vivo* techniques, which include transformation of cells *in vitro* with naked DNA, DNA encapsulated in liposomes, appropriate integration vectors followed by introduction into a host organ ("*ex vivo"* gene therapy).

Gene therapy provides a means for transfer of a desired gene into a subject with the subsequent *in vivo* expression thereof. Gene transfer can be accomplished by transfecting the subject's cells or tissues *ex vivo* and reintroducing the transformed material into the host. Alternatively, genes can be administered directly to the recipient.

Nabel et al., Science (1990) 249: 1285-1288, pertains to *in vivo* intra-arterial transfection of pigs with liposomes containing a β-gal expression plasmid. Site-specific gene expression was observed in the arterial wall. There are several drawbacks to *ex vivo* therapy. For example, if only differentiated, replicating cells are infected, the newly introduced gene function will be lost as those cells mature and die. *Ex vivo* approaches also can be used to transfect only a limited number of cells and cannot be used to transfect cells which are not first removed from the body.

As described above, in gene therapy, it is very important to appropriately select a gene to be introduced, target cells in which the introduced gene is to be expressed, gene transfer methods suitable for target tissues, and the administration route.

Cystic fibrosis (CF) is an autosomal recessive genetic disease causing inborn error of metabolism. CF patients are frequently found in the U.S. and Europe, and one in every 2, 000 to 2, 500 infants suffers from this disease. As a major symptom, abnormal external secretion produces viscous secreta, which are accumulated in organs such as lung, respiratory tracts, pancreas, liver, and small intestine. The current therapy of CF focuses on lung transplantation and antibiotic treatment of pulmonary infectious diseases, which is particularly fatal.

The causative gene of CF, cystic fibrosis transmembrane conductance regulator (CFTR) gene, has been identified (Riordan, J.R. et al., Science 245: 1066-1073, 1989), and it is expected to develop gene therapy for CF in which a vector carrying a normal CFTR gene is introduced to airway epithelia. In gene therapy for CF, the exogenous gene should be introduced *in vivo* because ex *vivo* treatment cannot be applied to lung and upper airway.

Several attempts have been made to administer vectors to lung. Hazinski et al. (Am. J. Respir. Cell Mol. Biol. (1991) 4: 206-209) discloses liposome-mediated gene transfer of DNA into the intact rodent lung. Cationic liposomes were complexed to three fusion gene constructs composed of 1) the chloramphenicol acetyltransferase (CAT) gene linked to a Rous sarcoma virus (RSV) promoter; 2) the CAT gene linked to a mouse mammary tumor virus (MMTV) promoter; and 3) a cytomegalovirus-β-galactosidase (CMV-β-gal) fusion gene. The liposome/DNA complexes were instilled into the cervical trachea of rats and detectable levels of gene expression observed.

Brigham et al. (Am. J. Med. Sci. (1989) 298: 278-281) describes the *in vivo* transfection of murine lungs with the CAT gene using a liposome vehicle. Transfection was accomplished by intravenous, intratracheal or intraperitoneal injection. Both intravenous and intratracheal administration resulted in the expression of the CAT gene in the lungs. However, intraperitoneal administration did not.

Canonico et al. (Clin. Res. (1991) 39: 219A) describes the expression of the human α-1 antitrypsin gene, driven by the CMV promoter, in cultured bovine lung epithelial cells. The gene was added to cells in culture using cationic liposomes. The experimenters also detected the presence of α-1 antitrypsin in histological sections of the lung of New Zealand white rabbits following the intravenous delivery of gene constructs complexed to liposomes.

Furthermore, U.S. Patent No. 5, 958, 893 discloses a method for introducing a gene encoding truncated CFTR using currently available vectors such as adenovirus vectors or cationic liposomes.

It was demonstrated, however, that adenovirus-mediated gene transfer to airway epithelia produced low gene transfer efficiency; low rate of uptake of adenoviral particles to the apical plasma membrane could be a cause of inefficient gene transfer, and lack of both the αβγ3 integrins and the CAR receptors which are the receptors for adenovirus, in apical surface of airway epithelial cells (Goldman, M. et al., Gene Ther. 3: 811-818, 1996, Boucher, R.C., J. Clin. Invest 103: 441-445, 1999). In the case of cationic liposomes, mucus reportedly prevented their uptake, and gene transfer efficiency was improved by removal of the mucus (Kitson, C. et al., Gene Ther. 6: 534-546, 1999, Zabner, J. et al., J. Biol. Chem. 270: 18997-19007, 1995, Fasbender, A. et al., Gene Ther. 4: 1173-1180, 1997).

To date, no report is available for vector systems and gene transfer methods enabling efficient introduction of exogenous genes to airway epithelia. It has thus been desired to develop vectors for efficient gene transfer to airway epithelia.

Sendai virus belonging to the family Paramyxoviridae is very useful as a vector for gene transfer, and its development is in progress (Kato, A. et al., EMBO J. 16: 578-598, 1997, WO97/16538, WO97/16539). Sendai virus shows low toxicity and expresses genes introduced therein at an extremely high level. This virus is also very safe because a gene insert in the virus vector is never integrated into the host chromosome. It has been reported that transfection ability of a Sendai virus vector is different from that of adenovirus (Goldman, M. et al., Gene Ther. 3: 811-818, 1996, Boucher, R.C., J. Clin. Invest 103: 441-445, 1999). For example, adenovirus is likely to infect injured sites, compared to uninjured sites (Kitson, C. et al., Gene Ther. 6: 534-546, 1999, Zabner, J. et al., J. Biol. Chem. 270: 18997-19007, 1995, Fasbender, A. et al., Gene Ther. 4: 1173-1180, 1997). These reports suggest that Sendai virus can complement the defect of adenovirus.

### Disclosure of the Invention

An objective of the present invention is to provide a composition comprising a vector suitable for introducing exogenous genes to airway epithelia and suitable for a method for introducing exogenous genes using the vector.

The present inventors investigated *in vitro* and *in vivo* gene transfer efficiency of a recombinant Sendai virus vector, adenovirus vector, and cationic lipid complex, each containing an exogenous gene, to airway epithelial cells derived from various animals. The results showed that the Sendai virus vector much more efficiently introduced the exogenous gene to airway epithelial cells than the adenovirus vector and cationic lipid complex.

The inventors also found that the recombinant Sendai virus vector efficiently introduced the exogenous genes not only to permissive mouse respiratory tracts, but also to non-permissive airway epithelial cells of large animals such as ferret, sheep, and human. Furthermore, the Sendai virus vector was found to infect submucosal glands as well as apical surfaces of epithelial cells. Based on these findings, the present invention was completed.

Specifically, the present invention provides a composition for introducing exogenous genes to airway epithelia comprising a recombinant Sendai virus vector carrying an exogenous gene.

The present invention also provides composition suitable for a method for introducing exogenous genes to airway epithelia, the method comprising contacting a composition comprising a recombinant Sendai virus vector carrying an exogenous gene with airway epithelia covered with mucus.

The present invention will be illustrated below in more detail.

The "recombinant Sendai virus vector" used herein means a reconstitution product of virus and virus-like particles from recombinant Sendai virus cDNA and comprises recombinant Sendai virus RNA and a Sendai virus body having infectivity. The term "infectivity" used herein means the capability of a virus to transfer its nucleic acid, etc. into cells through its adhesiveness to the cells and penetrating capability into cells via various mechanisms including fusion of the viral membrane and host cellular membrane. The recombinant Sendai virus vector can be ribonucleoprotein (RNP).

The "gene" used herein includes RNA and cDNA.

The "airway epithelial cells" means pseudostratified ciliated epithelial cells, as well as goblet and Clara cells, present on the internal surface of airways of nose, pharynx, trachea, or any conducting airway, or cells present on gas-exchange alveolar surface including type-I and II pneumocytes in lung.

The recombinant Sendai virus vector of the present invention carries a recombinant Sendai virus gene. The native Sendai virus genome consists of short 3' leader region, nucleocapsid (N) gene, phospho (P) gene, matrix (M) gene, fusion (F) gene, hemaglutinin-neuraminidase (HN) gene, large (L) gene, and short 5' trailer region in this order.

The Sendai virus gene used as a starting material for producing the recombinant Sendai virus vector can be modified by deletion or substitution as long as the reconstituted recombinant Sendai virus vector can infect airway epithelial cells and express, in the infected cells, the exogenous gene that the vector carries. For example, incomplete viruses such as DI particles (J. Virol. 68: 8413-8417, 1994) can be used.

For used in gene therapy, the preferable recombinant Sendai virus vector has infectivity but is deficient in disseminative capability. Disseminative capability can be eliminated by deleting at least one of F gene, HN gene, and M gene. Such a vector includes, for example, the gene of Sendai virus Z strain deficient only in the F gene. Additional examples are pSeV18⁺b (+) (Yu, D. et al., Genes to Cells 2: 457-466, 1997) and pSeV(+) (Kato, A. et al., EMBO J. 16: 578-587, 1997).

The recombinant Sendai virus gene can be obtained by inserting an exogenous gene into the Sendai virus gene as described above. Any exogenous gene can be used as long as it encodes a protein to be expressed in target airway epithelial cells. For gene therapy for CF, CFTR gene (Riordan, J.R. et al., Science 245: 1066-1073, 1989), a causative gene of CF, can be used. The exogenous gene includes genes encoding naturally occurring proteins and genes obtained by modifying the above genes by deletion, substitution, or insertion and encoding proteins functionally equivalent to the naturally occurring ones. For example, U.S. Patent No. 5,958,893 discloses a modified CFTR gene. Examples of the other exogenous genes include genes encoding α-1 antitrypsin (Long et al., Biochem 23: 4828-2837, 1984) DNase, superoxidedismutase (SOD), catalase, etc.

The recombinant Sendai virus vector carrying an exogenous gene can be prepared, for example, as described below, referring to the methods of Kato, A. et al. (EMBO J. 16: 578-587, 1997) and Yu, D. et al. (Genes to Cells 2: 457-466, 1997).

First, a DNA sample containing a cDNA base sequence of a desired gene is prepared. Preferably, the DNA sample can be electrophoretically recognizable as a single plasmid at the concentration of 25 ng/µl or higher. NotI recognition site in the target cDNA sequence should be removed in advance if it exists. Forward and reverse (antisense strand) side synthetic DNA sequences are prepared as a primer pair containing the NotI recognition enzyme cleavage site sequence; the below-mentioned transcription termination sequences (E), intervening sequence (I), and transcription start sequence (S); and a part of the target gene sequence, to amplify and recover the desired gene fragment from the sample.

As a forward side synthetic DNA sequence, optional two or more oligo DNAs are selected from the 5' side, preferably four bases free of the NotI recognition site-derived sequences, GCG and GCC, more preferably ACTT, with adding to the 3' side the NotI recognition site gcggccgc, and optional nine bases with or without a multiple of six bases as a spacer sequence. Furthermore, a sequence corresponding to 25 bases of ORF from start codon ATG of the desired cDNA, including ATG, is added to the 3' side. In this case, approximately 25 bases are selected from the desired cDNA so that the 3' end of the forward side synthetic oligo DNAs should be G or C.

As a reverse side synthetic DNA sequence, optional two or more oligo DNAs are selected from the 5' side, preferably four bases free of the NotI recognition site-derived sequences, GCG and GCC, more preferably ATCC, with adding to the 3' side the NotI recognition site gcggccgc, and oligo DNAs of an insert fragment for adjusting the length. The length of this oligo DNAs is designed so that the total number of the complementary strand bases of cDNA and EIS bases derived from Sendai virus genome, including the NotI recognition site gcggccgc, becomes a multiple of six (so-called "rule of 6"; Kolakofski, D. et al., J. Virol. 72: 891-899, 1998, Calain, P. and Roux, L., J. Virol. 67: 4822-4830, 1993). The 3' end of the reverse side synthetic oligo DNAs is prepared by adding to the 3' side of the insert fragment the complementary strand sequence of S sequence of Sendai virus, preferably 5'-CTTTCACCCT-3', I sequence, preferably 5'-AAG-3', and the complementary strand sequence of E sequence, preferably 5'-TTTTTCTTACTACGG-3', a complementary sequence ending in either G or C, corresponding to 25 bases reversibly counted from a stop codon of the desired cDNA sequence.

The standard method using ExTaq polymerase (Takara Shuzo Co.) can be used for PCR. Preferably, Vent polymerase (NEB) is used and an amplified target fragment is digested by NotI to be inserted into the NotI site of plasmid vector pBluescript. A base sequence of the resulting PCR product is confirmed by a sequencer to select plasmids with a correct sequence. The selected plasmid is inserted into NotI site of a genomic cDNA plasmid of Sendai virus, such as pSeV18+b(+) (Yu, D. et al., Genes to Cells 2: 457-466, 1997) or pSeV(+) (Kato, A. et al., EMBO J. 16; 578-587, 1997), cleaved by NotI, to obtain recombinant Sendai virus cDNA to which an exogenous cDNA is inserted. Alternatively, the recombinant Sendai virus cDNA can be obtained by directly inserting into the NotI site without using plasmid vector pBluescript.

A recombinant virus vector can be obtained by transcribing the recombinant Sendai virus cDNA prepared as described above in vitro or in cells to reconstitute the virus. A virus can be reconstituted from cDNA by the known method (WO97/16538, WO97/ 16539).

Reconstitution from cDNA can be performed as follows.

Monkey kidney derived cell line LLCMK2 is cultured to be 70% to 80% confluent (1 x 10⁶ cells) in minimum essential medium (MEM) containing 10% fetal calf serum (FCS) and antibiotics (100 units/ml penicillin G and 100 µg/ml streptomycin) on a 6-well plastic plate. The cells are then infected with recombinant vaccinia virus vTF7-3 expressing T7 polymerase, which is inactivated by UV irradiation (Fuerst, T. R. et al., Proc. Natl. Acad. Sci. USA 83: 8122-8126, 1986, Kato, A. et al., Genes Cells 1: 569-579, 1996), by 2 PFU/cell. One hour after the infection, the cells were further cotransfected with 60 to 2 µg, more preferably 3 to 5 µg, of the above recombinant Sendai virus cDNA and the plasmid expressing viral proteins which act trans essential for the synthesis of whole Sendai virus gemone (24 to 0.5 µg of pGEM-N, 12 to 0.25 µg of pGEM-P, and 24 to 0.5 µg of pGEM-L, more preferably, 1 µg of pGEM-N, 0.5 µg of pGEM-P and 1 µg of pGEM-L) (Kato, A. et al., Genes Cells 1: 569-579, 1996) by the transfection method such as the lipofection method using Superfect (QIAGEN Inc.). The transfected cells are cultured in serum-free MEM containing 100 µg/ml of rifampicin (Sigma) and cytosine arabinoside (AraC), more preferably, 40 µg/ml of cytosine arabinoside (AraC) to determine an optimal concentration of these drugs so as to minimize cytotoxicity of vaccinia virus and maximize the recovery of the virus (Kato, A et al., 1996, Genes Cells 1: 569-579). Forty-eight hours after the transfection, the cells are recovered and disrupted by repeating freeze thaw three times, and injected into chorioallantoic cavity of 10-day embryonated chicken egg. After three days, the chorioallantoic fluid is recovered to determine the virus titer by measuring hemagglutinin activity (HA). HA can be determined by "endo-point dilution method" (Kato, A. et al., 1996, Genes Cells 1: 569 579). The samples from which HA has not been detected are further injected into embryonated chicken eggs. The titer of Sendai virus to be recovered is usually 10⁸ to 10⁹ PFU/ml and that of the vaccinia virus vTF7-3 contained together is 10³ to 10⁴ PFU/ml or lower. The samples are diluted 10⁶ fold and multiplied again in chicken eggs to remove the vaccinia virus. The recombinant viruses obtained through the second or third passage in the embryonated chicken eggs are stored to obtain recombinant virus vectors into which the desired cDNA is inserted. Plaque forming potential of the stored virus is generally 10⁹ PFU/ml or 10,240 HA unit/ml, and this value will be kept if the virus is stored at -80°C.

Host cells used for reconstitution are not particularly limited as long as the recombinant Sendai virus cDNA can reconstitute in the cells. Cell lines used as hosts includes cultured cells such as CV-1 cells derived from monkey kidney and BHK cells derived from hamster kidney as well as LLCMK2 cells, and cells of human origin.

The reconstituted recombinant Sendai virus can be bound to adhering molecule, ligand, receptors, etc. on its envelope surface for facilitating the adherence to specific cells.

The above-described chorioallantoic fluid containing the virus vector can be used as the composition comprising the recombinant Sendai virus vector of the present invention.

The composition of the present invention can comprise any physiologically acceptable medium such as deionized water, 5% dextrose in water, and the like. Other auxiliary components maybe included in the composition such as stabilizers, biocides, etc. The composition comprising the recombinant Sendai virus vector can be in lyophilized dosage form. Such a composition can further comprise, in addition to the above-described auxiliaries, stabilizers such as albumin, Prionex^{™} (Pentapharm, Japan), or the like.

The exogenous gene contained in the recombinant Sendai virus can be introduced into airway epithelial cells by contacting the composition containing the recombinant Sendai virus vector with airway epithelial cells covered by mucus. When cationic lipid is used for gene transfer to airway epithelial cells, the airway mucus is a serious barrier to cationic lipid-mediated gene transfer and the mucus must be removed for introducing exogenous genes. In contrast, the composition containing the Sendai virus vector of the present invention can readily introduce exogenous genes by merely contacting it with airway epithelial cells with mucus.

The composition suitable for a method for introducing exogenous genes of the present invention can be used for gene therapy by expression of exogenous genes that is expected to treat the disorder of airway epithelial cells, or endogenous genes encoding proteins deficient in the cells. For example, the composition of the present invention containing the virus vector carrying the CFTR gene can be useful for therapy of CF. Gene therapy can be performed by applying the virus vector-containing composition of the present invention to airway epithelial cells of diseased sites *in vivo* or *ex vivo* and allowing exogenous genes to express in the cells. *In vivo* gene transfer can be carried out by local application such as instillation or inhalation using nebulizers to nasal cavity or lung. Examples of nebulizers include those commercially available and typically used in the treatment of asthma.

The virus vector-containing composition of the present invention suitable for the preparation of a medicament can be applied to any mammals including human, mouse, rabbit, sheep, bovine, monkey, etc.

### Brief Description of Drawings

Figure 1 shows *in vivo* gene transfer efficiency of the recombinant Sendai virus vector of the present invention and a cationic lipid complex in mouse lung and nose. Error bars indicate SEM.

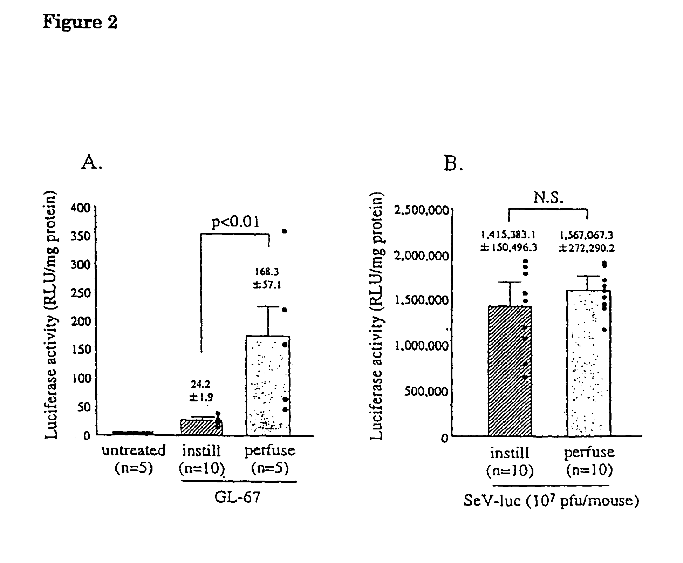

Figure 2 shows effect of contact time on gene transfer efficiency of the recombinant Sendai virus vector of the present invention (A) and a cationic lipid complex (B) in mouse nose assessed by nasal instillation (brief contact) and perfusion (longer contact). Error bars indicate SEM.
Figure 3 shows gene transfer efficiency of the recombinant Sendai virus vector of the present invention and the adenovirus vector in mouse nose assessed by nasal instillation. Error bars indicate SEM.
Figure 4 shows microscopic photographs detecting by X-gal staining β-gal gene expression in mouse bronchile, tranchea, and nose introduced by nasal instillation of the recombinant Sendai virus vector of the present invention and the adenovirus vector.
Figure 5 shows microscopic photographs detecting by X-gal staining gene expression in mouse tranchea and nose of β-gal introduced by nasal instillation of the recombinant Sendai virus vector of the present invention and the adenovirus vector. NC indicates non-ciliated secretory cells and BC basal cells.
Figure 6 shows gene expression of in ferret lung of β-gal introduced by nasal instillation of the recombinant Sendai virus vector of the present invention. R1 indicates lower right lobe and L1 upper left lobe.
Figure 7 shows microscopic photographs detecting gene expression of in ferret lung of β-gal introduced by nasal instillation of the recombinant Sendai virus vector of the present invention. Photgraph a is for upper left lobe, b mid right lobe, c submucosal glands, and d control. Furthermore, Lm indicates bronchial cavity and sm submucosal glands.
Figure 8 shows gene transfer efficiency of the recombinant Sendai virus vector of the present invention and a cationic lipid complex to human nasal epithelial cells collected from human healthy donors. Error bars indicate SEM.
Figure 9 shows gene transfer efficiencyof the recombinant Sendai virus vector of the present invention and a cationic lipid complex to sheep tracheal cells. F indicates fresh cells and MD mucus-depleated cells. Error bars indicate SEM.
Figure 10 shows gene transfer efficiency of the recombinant Sendai virus vector of the present invention and a cationic lipid complex to mucin-added sheep tracheal cells. F indicates fresh cells and MD mucus-depleted cells. Error bars indicate SEM.
Figure 11 shows gene transfer efficiency of the recombinant Sendai virus vector of the present invention and the adenovirus vector to edge and mid portions of sheep tracheal cells. Error bars indicate SEM.
Figure 12 shows microscopic photographs detecting signals of GFP introduced by the recombinant Sendai virus vector of the present invention and the adenovirus vector to sheep tracheal cells.
Figure 13 schematically shows a conventional whole-cell configuration.
Figure 14 shows the time course of forskolin-induced inward current at -60 mV in COS7 cells expressing sample-1 SeV/CFTR. The membrane potential was kept at a holding potential of -60 mV. The vertical deflection indicates the rectangular pulses (duration, 1 s) at 15 s intervals from -100 mV to +60 mV. The dash line indicates the zero current level.
Figure 15 shows effects of forskolin on the membrane current in COS7 cell expressing sample-1 SeV/CFTR. The membrane potential was kept at a holding of-60 mV. The dash line indicates the zero current level. Glibenclamide (300 µM) inhibited forskolin-induced Cl currents.
Figure 16 shows current-voltage relationships obtained in the absence or presence of 10 µM forskolin. The membrane current amplitude was measured as mean value of the last 100 ms of the command pulses (1 s duration). The line was fitted by the least squares method.
Figure 17 shows the net membrane current obtained by subtracting the membrane current recorded before the application of forskolin from that recorded during the application of 10 µM forskolin in COS7 cells expressing sample-1 SeV/CFTR. The membrane potential was kept at a holding potential of -60 mV. The dash line indicates the zero current level.

### Best Mode for Carrying out the Invention

The present invention will be illustrated with reference to the following examples, but is not construed as being limited thereto.

### Example 1

### Construction and reconstitution of recombinant Sendai virus vector

A recombinant Sendai virus was constructed by the known method (Kato, A. et al., EMBO J. 16: 578-598, 1997, Hasan, M.K. et al., J. Gen. Verol. 78: 2813-2810, 1997). First, 18 bp of spacer sequence (5'-(G)-CGGCCGCAGATCTTCACG-3') with the NotI restriction site was inserted into the proximal locus between the leader sequence and the 5'-end of the sequence encoding N-protein of cloned SeV genomic cDNA, pSeV (+), to obtain plasmid pSeV18⁺b (+), which also contains a self-cleaving ribozyme site from antigenomic strand of hepatitis delta virus. Whole cDNA of *E. coli* lacZ containing nuclear localising signal, luciferase, green fluorescent protein (GFP), and *E*. *coli* lacZ were amplified by polymerase chain reaction using the primers with the NotI site and new sets of SeV E and S signal sequence-tags for exogenous genes, and inserted into the NotI site of the cloned genome. The whole length of templete SeV gemones with exogenous genes were arranged to multiple of six nucleotides. Template SeV genome with exogenous gene, plasmids encoding N-, P- and L-proteins (pGEM-N, pGEM-P, pGEM-L) were complexed with commercially available cationic lipids, GL-67-DOPE-PEG (Genzyme Co. Ltd.) and co-transfected with vaccinia virus vT7-3 (Fuerst, T.R. et al., Proc. Natl. Acad. Sci. USA 83: 8122-8126, 1986, Kato, A. et al., Genes Cells 1: 569-579, 1996) to LLCMK2 cells. Fourty hours later, the cells were disrupted by 3-cycles of freezing and thawing, and injected into the chorioallantoic cavity of 10-day-old embryonated chicken eggs. Then the virus was recovered and the vaccinia virus was eliminated by second passage in eggs. Virus titer was determined by hemagglutination assay (HA) (Kato, A. et al., Genes Cells 1: 569-579, 1996) using chicken red blood cells, and the chorioallantoic fluid containing the viruses were kept freeze at -80°C just before use to serve as the composition containing the recombinant Sendai virus vector of the present invention.

### Example 2

### In vivo gene transfer to the mouse nose and lung by nasal instillation or nasal perfusion

### 2-1. Comparing Sendai virus vector with cationic lipid

pCMV-luciferase was constructed by insertion of HindIII-BamHI fragment of pGL3-control vector (Promega), into the multicloning site of pcDNA3 (Invitrogen) to be driven by human cytomegalovirus immediate early (CMV-IE) promoter. pCMV-luciferase was then complexed with GL-67-DOPE-PEG (Genzyme Co. Ltd.) to obtain GL-67-pCMV-luc.

To examine gene transfer efficiency of the vectors to the lung and the effect of contact time on gene transfer efficiency, the vectors were administered to nasal cavity by nasal instillation and nasal perfusion. First, male balb/c mice (6-8 weeks) were instilled intranasally with 100 µl of various concentration of the Sendai virus vector containing luciferase (SeV-luc) prepared in Example 1 or GL-67-pCMV-luc (80 µg DNA/mouse) by the known method (Yonemitsu, Y. et al., Gene Ther. 4: 631-638, 1997).

Nasal perfusion was performed by intranasally inserting 5 mm of a catheter, and perfusing 150 µl each of vector solution at rate of 5 to 6 µl/minutes using Peristaltic pump (model P-1, Pharmacia Biotech). Two days after gene transfer, the mice were killed under the sufficient anesthesia by intraperitoneal injection of overdose pentobarbitar, and turbinates, trachea, and lung were harvested and subjected to luciferase assay.

As a control, pSeV18b+ used in Example 1 was subjected to the same gene transfer procedure as described above. This plasmid was used as a control in the following examples.

Luciferase assay was performed as follows according to the known method (Yonemitsu, Y. et al., Gene Ther. 4: 631-638, 1997). First, tissues were washed with PBS and minced with scissors in the 1 x lysis buffer with protease inhibitor cocktail, cetrifuged at 13, 000 rpm for 10 minutes at 4°C and 30 µl of the supernatant was subjected to 100 µl of luciferase assay buffer (Promega). The light intensity was measured by Turner TD20e luminometer (Turner Co.) with 10 seconds integration soon after 10 seconds preincubation at 20°C. In this condition, 1 pg of recombinant luciferase (Promega) is equivalent to 2.56 x 10¹ RLU. The protein concentration was measured by Bradford's method using commercially available protein assay system (Bio-Rad Laboratories Ltd., Hertfordshire, UK) according to standard curve correspond to bovine serum albmin. The data was expressed as RLU/mg protein, and each samples were measured more than twice.

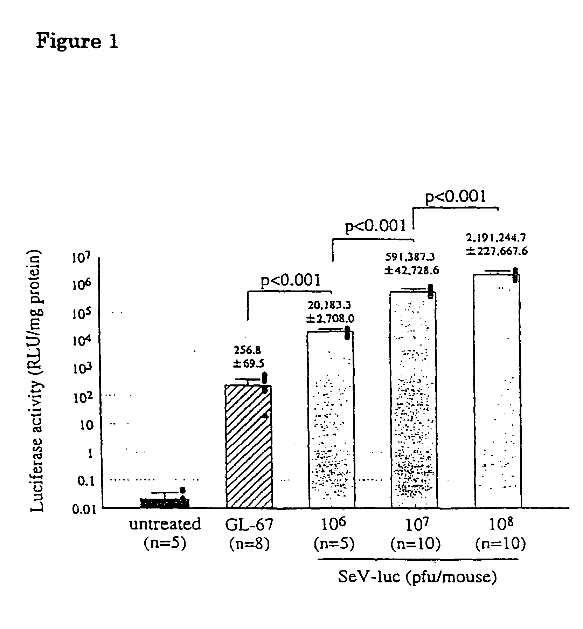

Figures 1 (lung) and 2 (nose) show comparison of gene transfer efficiency between SeV-luc and GL-67-p-CMV-luc. As shown in Fig. 1, SeV-luc transfected lungs exhibited more than 1,000-fold higher luciferase activity than that of GL-67-pCMV-luc dose-dependently. Luciferase gene expression by SeV showed approximately 10,000-times greater than GL-67-pCMV-luc without significant difference between different contact time. These results suggest that the SeV vector enables efficient gene transfer to mouse lung and nose by merely contacting the vector with airway epithelia.

### 2-2. Comparing Sendai virus vector with adenovirus vector

SeV-luc or adenovirus vector containing a luciferase gene, AdCMV-luciferase (Ade-luc) (Kendall, J.M. et al., Cell Calcium 19: 133-142, 1996) was instilled intranasally in the same manner as in Example 1, turbinates, trachea, and lung were harvested and subjected to luciferase assay.

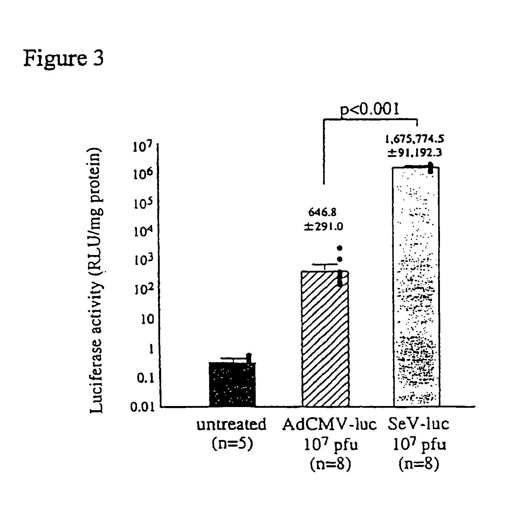

Sendai virus vector and adenovirus vector both carrying lacZ gene with nuclear localizing signal of simian virus large T antigen (SeV-NLS-lacZ and AdCMV-nls-lacZ) were prepared and subjected to nasal instillation in the same manner as described in 2-1. Bronchi, tracheae, and turbinates were harvested. Each tissue was fixed with ice-cooled 2% paraformaldehyde with 0.25% glutaraldehyde in 0.1M PBS for 10 minutes and followed by X-gal staining (solution: 5 mM potassium ferrous cyanide, 5 mM ferric cyanide, 2 mM magnesium chloride, 1 mg/ml 5-bromo-4-chloro-3-indolyl-β-D-galacto-pyranoside) for 3 hours at room temperature under rotate shaker. The X-gal stained tissue was refixed and mounted to paraffin, and 5 µm sections were examined under light microscope. The results are shown in Figs. 3, 4, and 5.

As shown in Fig. 3, SeV-luc transfected cells demonstrated 5,000-times greater gene expression than that of Ade-luc.

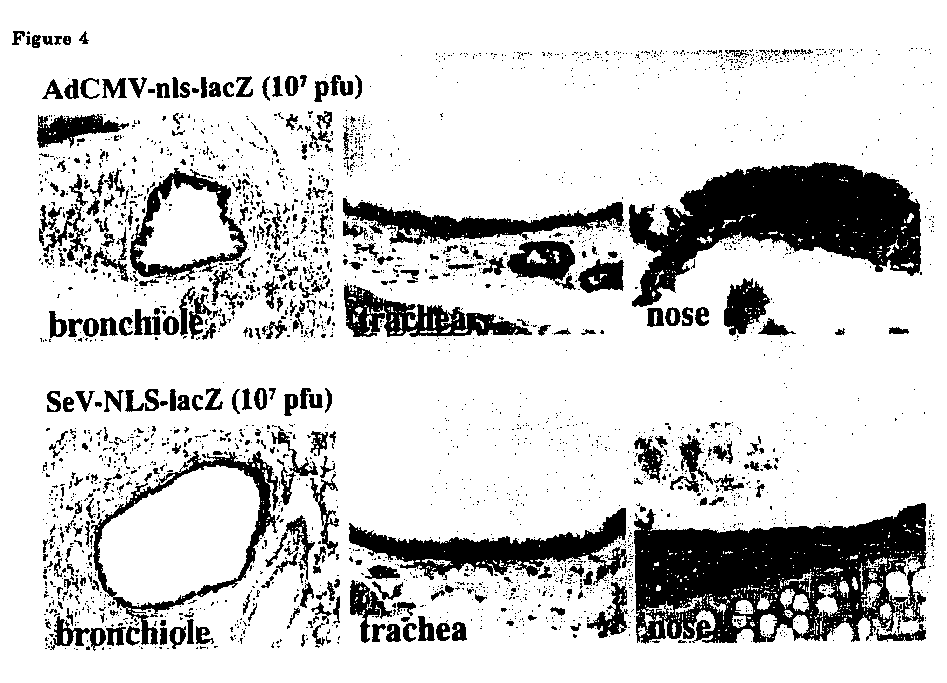

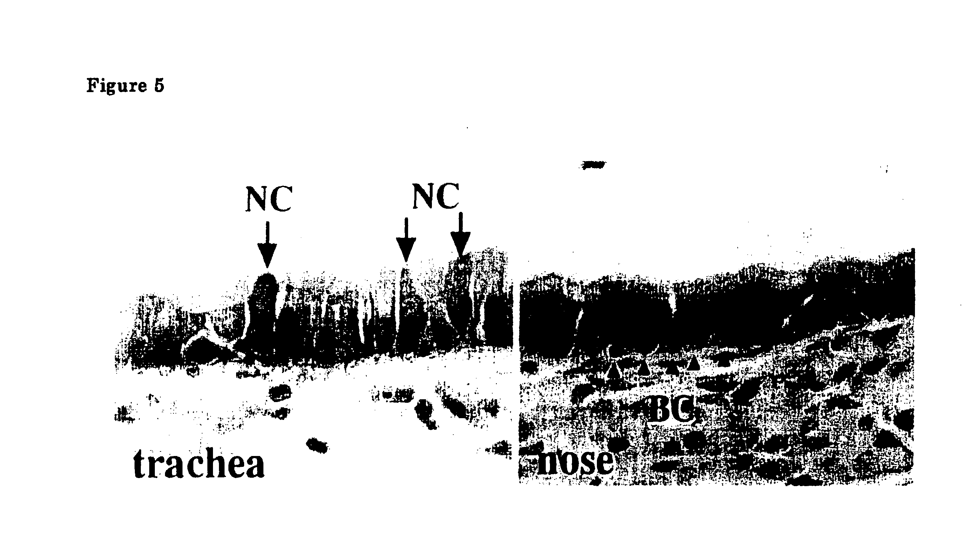

X-gal positive epithelial cells were scattered in the bronchioli in similar frequency in both vector innoculation (Fig. 4). On the other hand, X-gal positive cells were frequently observed in SeV-NLS-lacZ treated animals, while blue cells were rare in the trachea or nose of AdCMV-nls-lacZ treated mice. As shown in Fig. 5, blue stains were seen not only in ciliated columnar cells but also non-ciliated secretary cells (NC). In contrast, no detectable blue signals were seen in basal cells (BC).

These results reveal that Sendai virus vectors enable gene transfer to airway epithelial cells to which adenovirus vectors cannot introduce genes.

### Example 3

### Gene transfer to lung of ferret

Ferrets (500-600 g weight) were anaesthetised and instilled intranasally with 3 ml of purified SeV-LacZ in BSS with either 3 x 10⁸ or 3 x 10⁹ pfu/ml (n=3 each group), as in Example 2. Controls (n=2) received 3 ml of SeV-Luc (10⁹ pfu/ml). Forty-eight hours post-infection, ferrets were sacrificed, the trachea cannulated in situ and the lungs inflated with ice cold fixative solution (2% formalin, 0.2% glutaraldehyde, 2 mMMgCl₂, 5 mM EGTA in PBS, pH 7.3). The trachea and lungs were excised en bloc and underwent X-Gal staining as described in Example 2. Each lung was dissected into 7 parts: trachea, 4 right lobes (upper (R1), mid (R2, R3), and lower (R4)) and 2 left lobes (upper (L1) and lower (L2)), and β-gal positive cells in the airway epithelia and submucosal glands were quantified microscopically by point counting using a graticulated lens. Ten x20 magnification fields/airway were assessed to obtain the percentage of blue cells/airway and 3 to 8 airways randomly taken from different regions of a lobe (proximal, medium and distal) were assessed for each lobe. For submucosal glands, 10 to 28 fields (containing at least 4 glands) /lobewereassessed. The error of repeat measurement (ERM) expressed as a coefficient of variation (CV) was 18%. Intra-animal CV was between 24 and 43% for animal receiving 10⁸ pfu/ml and between 8 and 14% for animals receiving 10⁹ pfu/ml.

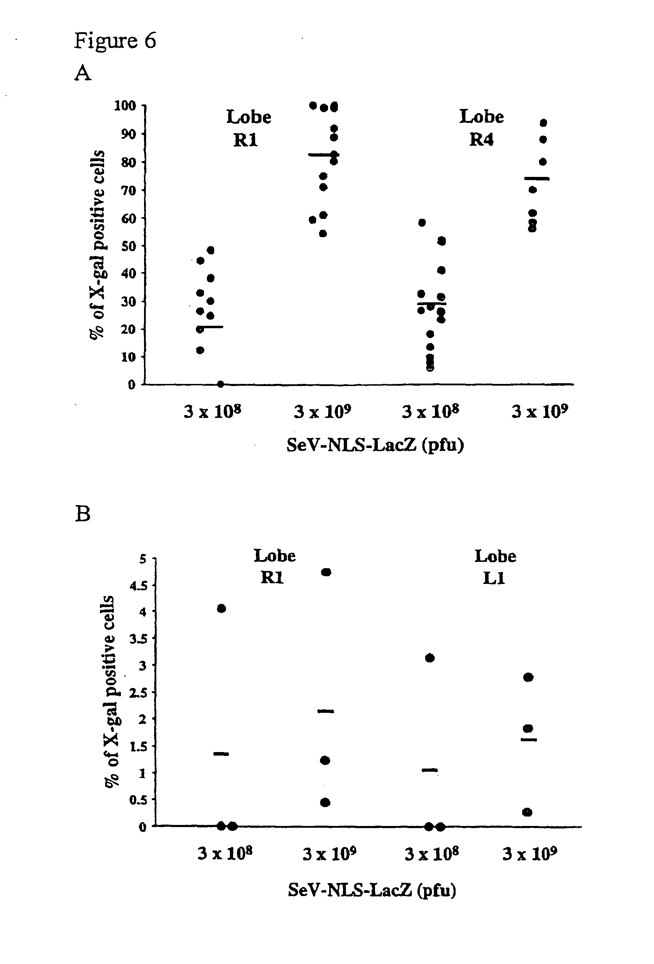

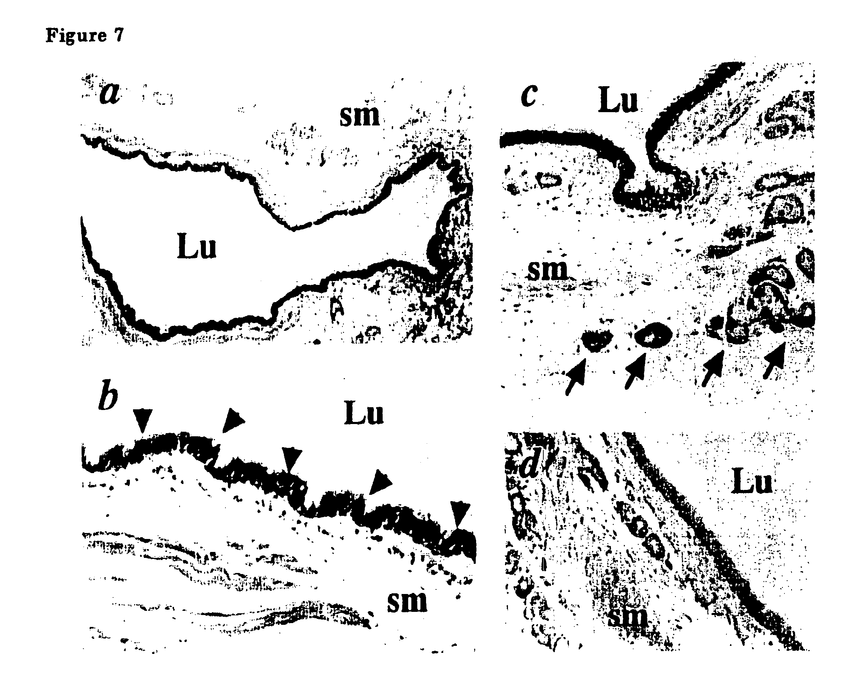

The airway epithelia (Fig. 6A and Fig. 7a and b) and submucosal glands (Fig. 6B and Fig. 7c) were exhibited β-galactosidase activity dose-dependently. Submucosal glands are the predominant sites of CFTR expression. No activity was found in control (Fig. 7d).

### Example 4

### Gene transfer to nasal epithelial cells from human healthy donors

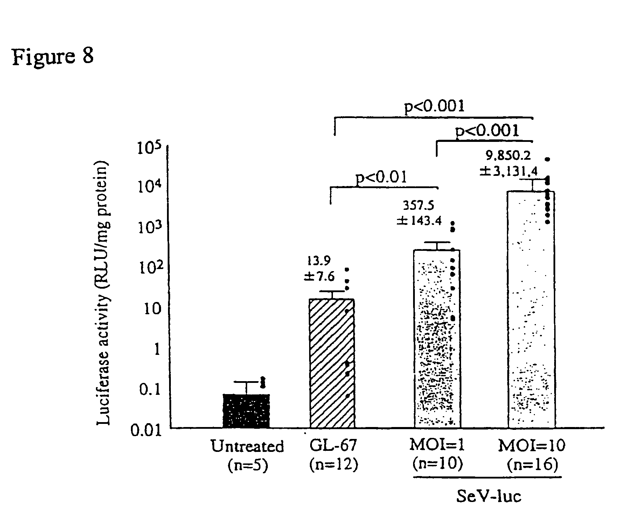

Nasal epithelial cells were collected by brushing from human healthy donors (6: male and 3: female). After 2-times wash with phosphate buffered saline (PBS: 137 mM NaCl, 3 mM KCl, 8 mM Na₂HPO₄, 1 mM KH₂PO₄, pH7 .2), the cells were resuspended in the culture medium (Dulbecco's modified Eagle's medium; DMEM) with 10% bovine fetal serum, divided into 2 or 3 groups, and placed in the each wells of 96-culture plate. The viability of the nasal cells were confirmed by phase-contrast microscopic observation of ciliary beating and microscopic count of trypan blue-positive cell numbers. Vector solutions (SeV-luc and GL-67-pCMV-luc) were added to each well. Twenty four hours later, the cells were collected, washed 3-times with PBS, and subjected to luciferase assay as described in Example 2. The results are shown in Fig. 8.

SeV-luc transfected cells demonstrated about 1, 000 times greater luciferase activity than that of GL-67-pCMV-luc transfected cells.

### Example 5

### Gene transfer to the sheep tracheal epithelia

### 5-1. Effect of mucus on gene transfer

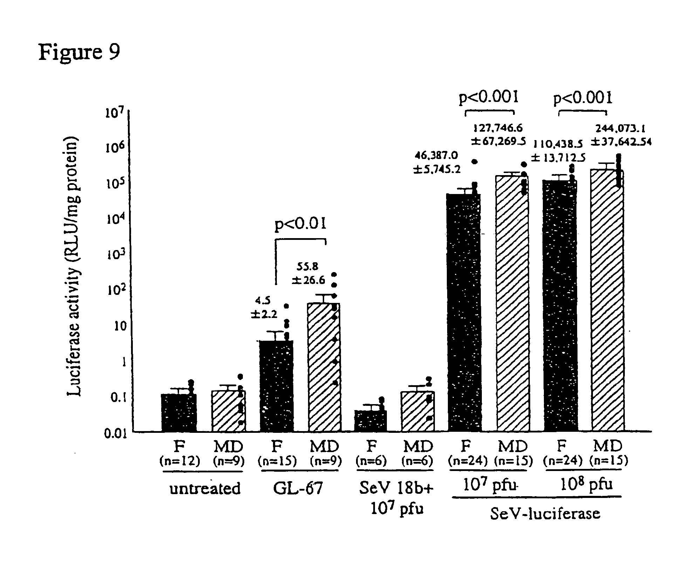

Effect of mucus on gene transfer efficiency of each vector was examined using a sheep tracheal strip model, which was prepared by a known method (Kitson, C. et al., Gene Ther. 6: 534-546, 1999). After killing, the epithelial layer of resected sheep trachea was dissected to muscle and adventitia, and was cut into 0.5 cm² square pieces subsequently confirmed the ciliary beating under the phase-contrast microscope. In some tissue, mucus depletion was followed to the known method (Kitson, C. et al., Gene Ther. 6: 534-546, 1999). These tissues were placed in the air-liquid interface. Ten µl of SeV-luc or GL-67-luc vector solution was applied to the apical surface to perform transfection. After 48 hours, the pieces were subjected to luciferase assay as described in Example 2. The results are shown in Fig. 9.

As shown in Fig. 9, mucus was not markedly affect SeV-mediated gene transfer compared to GL-67-luc-mediated gene transfer.

### 5-2. Effect of viscosity of mucus on gene transfer

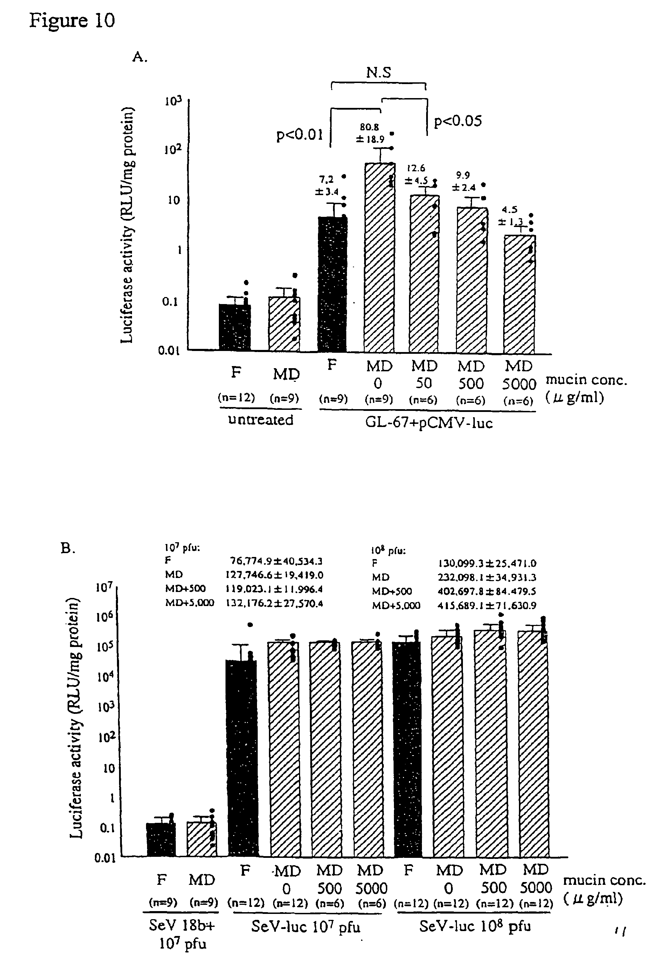

The procedure of 5-1 was repeated except that various concentrations of bovine salivary gland mucin were applied just before gene transfer. The results are shown in Fig. 10.

As Fig. 10 shows, gene transfer of GL-67-luc was inhibited by addition of mucin. Luciferase activities of the mucin added samples were not significantly different from that of fresh samples, suggesting barrier activity of the mucin, but not mucus viscosity, to cationic lipid-mediated gene transfer. On the other hand, serous mucin components do not affect SeV infection efficiency, while mucus viscosity mildly affect to SeV-mediated gene transfection.

### 5-3. Site-specific transfection efficiency

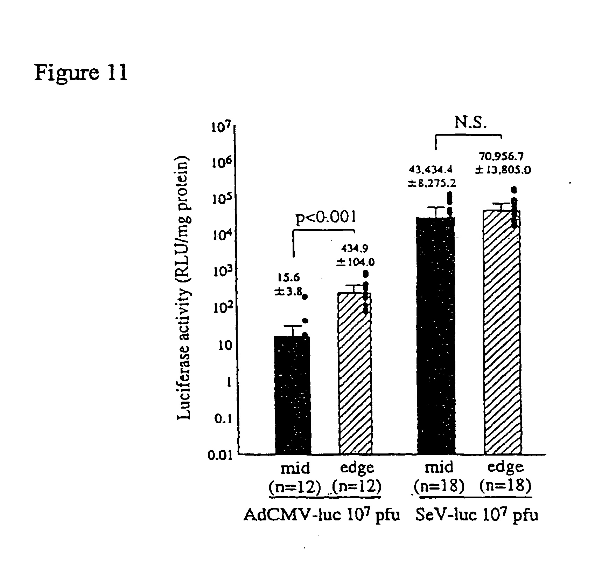

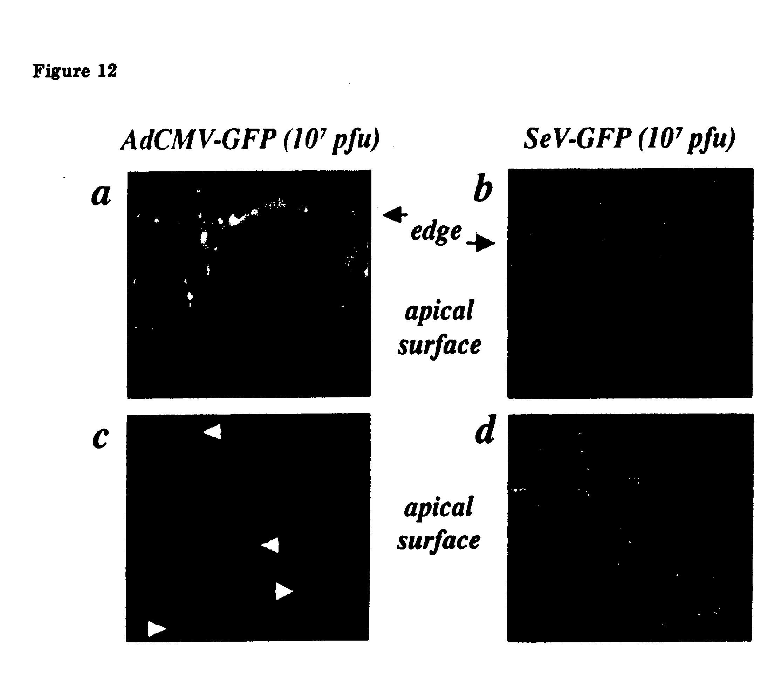

Sheep tracheal epithelia were transfected with SeV-luc or AdCMV-luc in the same manner as in 5-1. After gene transfer, the edge of the tissue was dissected and cut, and the luciferate activity of edge and mid portion was measured separately. The results are shown in Fig. 11. The same gene transfer procedure as above was repeated using SeV-GFP and high titer adenovirus serotype 5 carrying GFP driven by CMV-IE promoter, AdCMV-GFP (Kramel Biotech International Ltd.), in place of SeV-luc and AdCMV-luc. Two days after gene transfer, green fluorescent protein (GFP) signals were observed under fluorescent phase-contrast microscope. The results are shown in Fig. 12.

As shown in Figs. 11 and 12, AdCMV-luc showed higher expression in injured edge, while relatively little expression in uninjuredmid-portion of sheep tracheal tissue. In contrast, SeV-luc-treated tissue showed no significant difference in gene expression between edge and mid portions.

### Example 6

### Construction of SeV/CFTR and electrophysiological characterization

A recombinant Sendai virus vector expressing CFTR, the causative gene of CF, was constructed. CFTR gene (Riordan, J.R. et al., Science 245: 1066-1073, 1989) was amplified by PCR using a primer set containing E and S signal sequences. The primer set used are as follows.
Forward primer: 5'-acttgcggccgccaaagttcaatgcagaggtcgcctctg gaaaaggccagc-3' (SEQ ID NO: 4)
Backward primer: 5'-atccgcggccgcgatgaactttcaccctaagtttttct tactacggctaaagccttgtatcttgcacctcttcttc-3'(SEQ ID NO: 5).

The amplified fragment was inserted into the NotI site of pSeV18⁺b(+), and reconstitution of the virus was conducted as in Example 1.

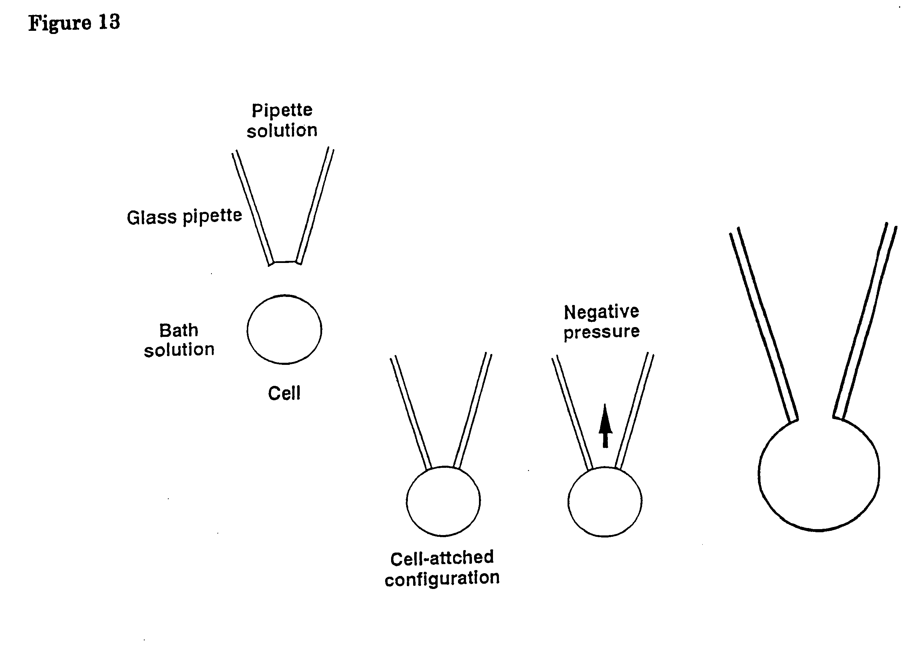

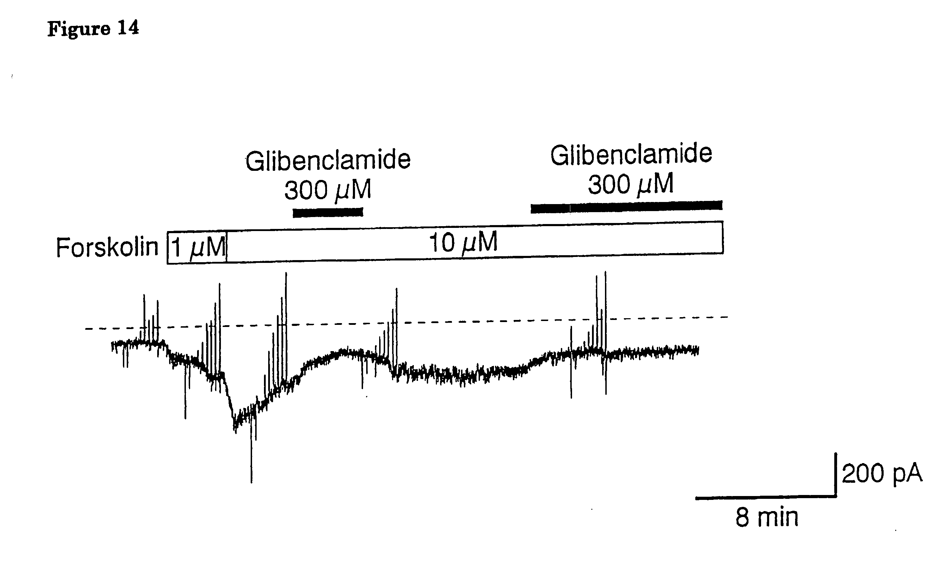

COS7cells were infected with the prepared CFTR-expressing Sendai virus (sample-1 SeV/CFTR), and the obtained infected cells were analyzed by the whole-cell patch clamp technique. Figure 13 shows a summary of the whole-cell patch clamp technique. A glass pipette containing a pipette solution was contacted with a cell within a bath solution, and negative pressure was applied to remove the cell membranes. In this occasion, the pipette solution contains 145 mM NMDG⁺, 148.4 mM Cl⁻, 6.7 mM Mg²⁺, 5 mMATP, 10 mM glucose, 0.1 mM EGTA, and 10 mM HEPES (titrated by Tris, pH 7.4), and the bath solution contains 141 mM Na⁺, 152.4 mM Cl⁻, 152.4 mM H₂PO₄⁻, 5 mM K⁺, 1.7 mM Mg²⁺, 2 mM Ca²⁺, 10 mM glucose, 0.1 mM EGTA, and 10 mM HEPES (titrated by Tris, pH 7.4). The effects of forskolin on the membrane current in COS7 cells expressing sample-1 SeV/CFTR were examined by whole-cell recording (Fig. 14). As a result, a forskolin concentration-dependent influx current was observed (a downward decrease of trace), which was suppressed (an upward transition) by glibenclamide (chloride channel blocker). The influx current was reproduced by adding forskolin again after a single wash, and was again suppressed by glibenclamide, which confirmed that the observed change in current was a specific drug-induced response.

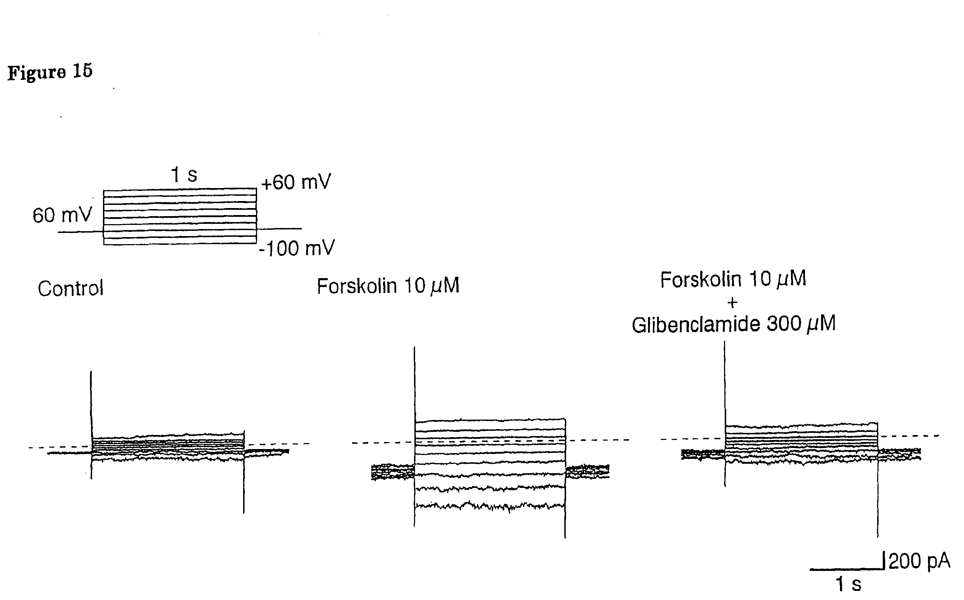

Next, the time-dependency of each drug-induced reaction was examined (Fig. 15). Forskolin induced a Cl⁻ current in COS7 cells expressing sample-1 SeV/CFTR, and a time-independent reaction characteristic to chloride channels was observed. Glibenclamide (300 µM) inhibited forskolin-induced Cl⁻ currents.

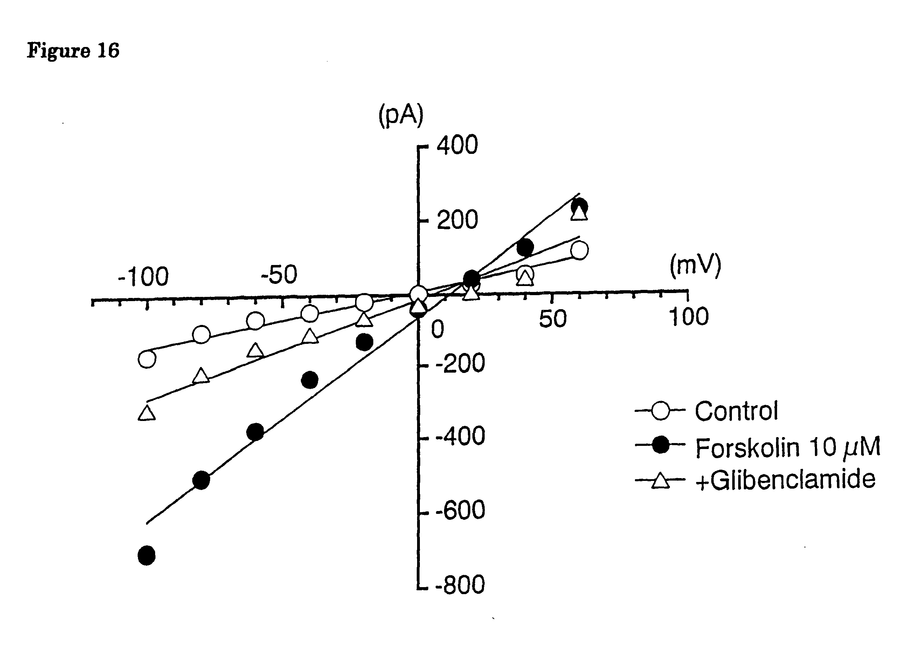

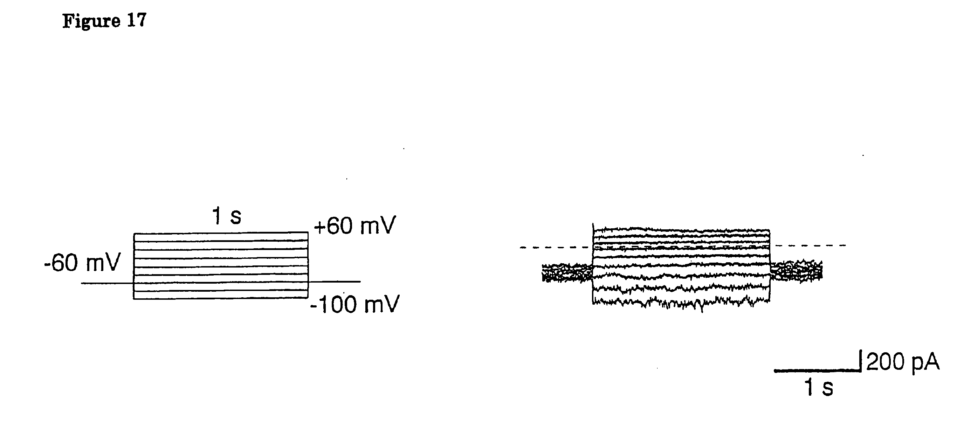

Figure 16 shows the current-voltage relationships derived based on the above data under the presence or absence of forskolin in COS7 cells expressing sample-1 SeV/CFTR. The lines cross at the point of origin if an endogenous Cl current is not present. In the graph obtained, the lines crossed between 10 and 20 mV. This suggests that a Cl current other than that induced by CFTR (forskolin-independent) is flowing in these COS7 cells. Figure 17 shows the difference in membarane current in the presence or absence of forskolin (net membrane current) obtained by subtracting the current recorded before the application of forskolin from that recorded during the application of forskolin.

### Industrial Applicability

The present invention provides a recombinant Sendai virus vector for introducing exogenous genes to airway epithelia, to which conventional vectors for gene transfer cannot introduce genes efficiently, and a composition suitable for a method for introducing exogenous genes using the vector. The recombinant Sendai virus vector of the present invention enables efficient gene transfer to native mucus-layered airway epithelial cells by briefly contacting the vector with the cells. The vector of the present invention can infect airway epithelial cells derived from mammals larger thanmice, which suggests that the vector of the present invention enables effective gene therapy in need of gene transfer to airway epithelial cells.

## Claims

1. A composition for introducing exogenous genes to airway epithelia comprising a recombinant Sendai virus vector containing an exogenous gene in its genome, wherein the exogenous gene is cystic fibrosis transmembrane conductance regulator (CFTR) gene or its deletion, substitution, or insertion derivative encoding a protein functionally equivalent to CFTR.

2. The composition according to claim 1, wherein the Sendai virus vector does not contain at least one of F gene, HN gene, and M gene.

3. The composition according to claim 1 or 2, wherein the composition further comprises chicken egg chorioallantoic fluid.

4. The composition according to any one of claims 1 to 3, wherein the composition is for treatment of cystic fibrosis.

5. Use of a recombinant Sendai virus vector containing an exogenous gene in its genome for the manufacture of a composition for gene therapy by introducing an exogenous gene to airway epithelia.

6. The use according to claim 5, wherein the Sendai virus vector does not contain at least one of F gene, HN gene, and M gene.

7. The use according to claim 5 or 6, wherein the composition further comprises chicken egg chorioallantoic fluid.

8. The use according to any one of claims 5 to 7, for the manufacture of a composition for treatment of cystic fibrosis.

9. The use according to any one of claims 5 to 8, wherein the exogenous gene is cystic fibrosis transmembrane conductance regulator (CFTR) gene or its derivative encoding a protein functionally equivalent to CFTR.

10. The use according to any one of claims 5 to 9, wherein the airway epithelia is present on nose, pharynx, trachea, or any conducting airway or gas-exchange surface in the lung.

## Patentansprüche

1. Zusammensetzung zur Einführung exogener Gene in Atemwegsepithelien, umfassend einen rekombinanten Sendaivirusvektor, welcher ein exogenes Gen in seinem Genom enthält, wobei das exogene Gen das Gen des zystische Fibrose-Transmembran-Leitfähigkeits-Regulators (CFTR) oder dessen Deletions-, Substitutions- oder Insertions-Derivat ist, welches ein Protein codiert, das funktionell äquivalent zu CFTR ist.

2. Zusammensetzung nach Anspruch 1, wobei der Sendaivirusvektor nicht mindestens eines der Gene F, HN und M enthält.

3. Zusammensetzung nach Anspruch 1 oder 2, wobei die Zusammensetzung zusätzlich eine chorioallantoische Flüssigkeit aus Hühnerei umfasst.

4. Zusammensetzung nach einem der Ansprüche 1 bis 3, wobei die Zusammensetzung zur Behandlung von zystischer Fibrose ist.

5. Verwendung eines rekombinanten Sendaivirusvektors, welcher ein exogenes Gen in seinem Genom enthält, zur Herstellung einer Zusammensetzung für die Gentherapie mittels Einführung eines exogenen Gens in Atemwegsepithelien.

6. Verwendung nach Anspruch 5, wobei der Sendaivirusvektor nicht mindestens eines der Gene F, HN und M enthält.

7. Verwendung nach Anspruch 5 oder 6, wobei die Zusammensetzung zusätzlich chorioallantoische Flüssigkeit aus Hühnerei umfasst.

8. Verwendung nach einem der Ansprüche 5 bis 7 zur Herstellung einer Zusammensetzung zur Behandlung von zystischer Fibrose.

9. Verwendung nach einem der Ansprüche 5 bis 8, wobei das exogene Gen das Gen des zystische Fibrose-Transmembran-Leitfähigkeits-Regulators (CFTR) oder dessen Derivat ist, welches ein Protein codiert, das funktionell äquivalent zu CFTR ist.

10. Verwendung nach einem der Ansprüche 5 bis 9, wobei die Atemwegsepithelien in der Nase, im Rachen, in der Luftröhre oder jedem leitenden Atemweg oder jeder Gasaustauschoberfläche in der Lunge anwesend sind.

## Revendications

1. Composition pour introduire des gènes exogènes dans l'épithélium des voies respiratoires comprenant un vecteur virus Sendai recombinant contenant un gène exogène dans son génome, dans laquelle le gène exogène est le gène régulateur de conductance transmembranaire de la mucoviscidose (CFTR) ou son dérivé de délétion, substitution ou insertion codant une protéine fonctionnellement équivalente à la CFTR.

2. Composition selon la revendication 1, dans laquelle le vecteur virus Sendai ne contient pas au moins l'un des gènes F, HN et M.

3. Composition selon la revendication 1 ou 2, dans laquelle la composition comprend en outre du fluide chorioallantoïque d'oeuf de poulet.

4. Composition selon l'une des revendications 1 à 3, dans laquelle la composition est pour le traitement de la mucoviscidose.

5. Utilisation d'un vecteur virus Sendai recombinant contenant un gène exogène dans son génome pour la préparation d'une composition pour la thérapie génique par introduction d'un gène exogène dans l'épithélium des voies respiratoires.

6. Utilisation selon la revendication 5, dans laquelle le vecteur virus Sendai ne contient pas au moins l'un des gènes F, HN et M.

7. Utilisation selon la revendication 5 ou 6, dans laquelle la composition comprend en outre du fluide chorioallantoïque d'oeuf de poulet.

8. Utilisation selon l'une des revendications 5 à 7, pour la préparation d'une composition pour le traitement de la mucoviscidose.

9. Utilisation selon l'une des revendications 5 à 8, dans laquelle le gène exogène est le gène régulateur de conductance transmembranaire de la mucoviscidose (CFTR) ou son dérivé codant une protéine fonctionnellement équivalente à la CFTR.

10. Utilisation selon l'une des revendications 5 à 9, dans laquelle l'épithélium des voies respiratoires est présent sur le nez, le pharynx, la trachée ou toute surface conductrice des voies respiratoire ou d'échange gazeux dans le poumon.
